# EUROPEAN PATENT SPECIFICATION

(11) **EP 3 318 225 B1**
(45) Date of publication and mention of the grant of the patent: **05.06.2019**
(21) Application number: 17200132.3
(22) Date of filing: 06.11.2017
(51) Int. Cl.: A61F 5/44, A61F 5/449, A61F 5/448

(54) **OSTOMY POUCH ARRANGEMENT**
OSTOMIEBEUTELANORDNUNG
DISPOSITIF DE POCHE POUR STOMIE

(30) Priority: 04.11.2016 NL 2017725
(43) Date of publication of application: 09.05.2018
(73) Proprietor: Tomas B.V., 5993 SC Maasbree (NL)
(72) Inventor: Gommans, Franciscus Petrus Johannes, 5993 SC Maasbree (NL)
(74) Representative: van der Krans, Arie

(56) References cited:
- WO-A2-01/35875
- US-A- 3 773 048
- US-A- 4 723 952
- US-A1- 2013 261 577
- US-B1- 7 935 097

## Description

### FIELD OF THE INVENTION

The invention relates to an ostomy pouch arrangement.

### BACKGROUND

Stoma patient find relief for intestinal excrements in ostomy pouch arrangements, which can be applied over the stoma and which capture such excrements. In the art ostomy pouch arrangements are known which employ an adhesive ring or annular element which is applied to the users abdominal wall, around the stoma. The ring is attached to a waste pouch, which can be drained via an outlet. Wearing comfort of such arrangements is limited, as the skin parts having the ring adhesively attached to the ostomy pouch ring can become irritated and inflamed, due to lack of oxygen. Moreover, adhesively attached ostomy pouch arrangements suffer from undesired loosening or leaking, as they do not have fastening and or support means for securing the arrangement to the subject's abdominal wall. This limits freedom of movement of the subject wearing the ostomy pouch arrangement.

Alternative solutions for adhesively attached ostomy pouch arrangement are known for example from published patent application GB2476680 A, wherein an adapter is applied to the subject's abdominal wall, having an opening for receiving the stoma . The adapter is connected to a wearing belt, which is worn around the subject's waist. The adapter of GB2476680 has a the stoma receiving opening, which is pressed against the subject's abdominal wall. This may cause discomfort, as the rim of this opening presses against the subject's abdominal wall in a sensitive place and provide only a single barrier for preventing leakage of fluids from the stoma. Subjects having a stoma are prone to abdominal hernia. A single pressure point may enhance a chance of suffering such hernia.

In international patent application WO0135875, which is regarded as the closest prior art, a colostomy apparatus for use in connection with a stoma is described characterized by a combination of the following elements: a) an absorbent pad having a centrally located hole which accommodates the stoma and which pad makes contact with the skin surrounding the stoma; a cup shaped stoma seal having a centrally located hole at its base to accommodate the stoma and an external sealing flange at its midsection; c) a base plate which comprises a ring shaped threaded collar having an inwardly projecting flange at the far end of the thread and a flexible skirt projecting out from the collar in the base plate; d) a utricle having a neck and a gas discharge passage forming a reservoir for waste matter from the stoma and; e) an utricular nut for sealing the utricle and having an external thread corresponding to the thread of the collar in the base plate; the arrangement being such that in use the cup shaped stoma seal is placed in the neck of the utricle and the reservoir section is fed through the utricular nut (4).

In US patent application number US2013261577A1 an ostomy belt is described with a main belt portion, a urine holding pouch portion and a stoma to pouch connector portion. The main belt portion has a semi rigid circular washer that forms an aperture toward one end of the belt. The urine holding pouch portion includes a rigid connector receiving ring, and includes a left and right hook type fastening strips attached to the inside surface of the urine pouch. The connector portion is made of low durometer silicone material and has an outwardly directed doughnut shaped ring capable of engaging a stoma in a secure leak proof and comfortable manner. An outer connector flange engages the circular washer of the belt. An inwardly facing flange engages the perimeter of the pouch connector ring. The main belt portion is wrapped around the mid section of a person and secured by the hook and loop fasteners.

Wearing belts and fastening means are usually narrow and uncomfortable for wearing and provide no protection against abdominal wall hernia, since they press in too small areas to the abdominal wall. Adapters may be uncomfortable since they can be incompatible and not adaptable to specific shapes and condition of individual subject's stoma's.

### SUMMARY

It is therefore an object of the invention to provide an ostomy pouch arrangement which overcomes the abovementioned disadvantages and which provides a leak free capture of stoma excrements, while preventing the abdominal wall hernia and providing wearing comfort for the subject wearing the ostomy pouch arrangement .

The object is achieved in an ostomy pouch arrangement, comprising an adapter, the adapter having a cup shaped body with an annular side wall and a baseplate. The baseplate of the adapter has an opening for receiving a stoma. The arrangement further comprises a belt connected to the adapter for wearing the arrangement on a subject's body. The adapter has an interface opposite the baseplate for connecting to an ostomy waste pouch inlet. The adapter is further provided with fastening means for tightening the belt around the subject's body to the adapter. The adapter has a continuous raised edge at a circumference of the baseplate in a direction facing the subject's abdominal wall.

The adapter is pressed by the belt against the subject's abdominal wall. The opening in the baseplate allows a stoma to protrude within the cup shaped body of the adapter. This way excrements from the stoma can be captured and directed to a waste pouch connected to the adapter. By the pressing, the continuous raised edge seals the space between the baseplate and the subject's abdominal wall. This prevents leaking from liquids beyond the raised edge and significantly enhances efficacy and wearing comfort of the ostomy pouch arrangement.

Moreover, the belt not only holds the adapter, but also provides support for the abdominal wall, thereby contributing to the prevention of the occurrence of hernia.

The connecting means comprise an annular element which is attachable to the adapter body at the side of the baseplate of the adapter facing the subject's abdominal wall. In this embodiment, the adapter has a flange at the outside of its side wall, and
the annular element is arranged for clamping an edge of the belt against the flange.

This allows the adapter to be mounted in an opening of the belt. In use, while wearing the belt, the belt presses against the abdominal wall, and with the adapter mounted in an opening of the belt, thus presses the adapter against the abdominal wall as well.

In an embodiment, the adapter body is provided with a screw thread, and annular element is provided with a screw thread corresponding with the screw thread of the adapter body.

This allows the adapter to be removably attached to the belt. This can be advantageous for cleaning of the belt and adapter separately. Moreover, when test fitting an ostomy pouch arrangement for a new patient, exchanging adapters is facilitated.

In an embodiment, the continuous raised edge is arranged at the annular element circumference facing the subject's abdominal wall. This allows an integrated function of the annular element in fastening the adapter to the belt in combination with the sealing function as described.

In an embodiment, the edge of the belt opening wherein the adapter can be fitted is thickened, and at least one of the annular element and the flange have an annular chamber for locking in the thickened edge of the belt opening.

This way the edge of the belt is not only clamped between the annular element and the flange, but also locked in, allowing a more secure fitting of the adapter in the belt.

In an embodiment, the baseplate of the adapter has a convex shape.

This allows the opening of the baseplate to be pressed into the abdominal wall, allowing a for example a more deeply located stoma to pass through the opening. This further enhances wearing comfort and prevents leakage for subject's having a compatible stoma with such a base plate shape.

In an embodiment, the baseplate of the adapter has a flat shape.

This relieves pressure around the stoma an allows an inverted stoma, in an inside-out configuration to pass through the opening. This enhances wearing comfort and prevents leakage for subject's having an inverted, inside-out stoma with such a base plate shape.

In an embodiment the belt has two ends, in use facing each other, wherein each end is provided with corresponding mutually cooperating fastening for fastening the two ends together.

The fastening means can be provided with length adjustment means such as straps for adjusting the belt length. As a base length of the belt can be chosen more permanently, this allows for size variations in a prolonged period of use, and provide wearing comfort for the user.

In an embodiment the adapter is positioned in proximity of the fastening means of the belt.

This causes the fastening means to be located near de abdomens wall, and not for instance on the subject's hip or back, as the adapter is normally located at the abdomens wall. This enhances the wearer's comfort when putting on the arrangement and fastening the fasteners.

In an embodiment one end of the belt has a freely positionable , detachable and re-attachable section on which the mutually cooperating fastening means are located. The detachable and re-attachable section can be positioned freely relative to each other, allowing belt length adjustment. Moreover an angle can be introduced between the belt main section and the detachable, re-attachable section, which enhances wearing comfort, especially for users having enlarged bellies. This applies especially when the lower side of the belt covers a smaller circumferential distance over the subject's belly than the upper side of the belt.

In an embodiment, the detachable and re-attachable section is attachable to the belt using a hook and loop connection.

In an embodiment, the belt is provided with ribs, which ribs extend in a direction transverse to an end to end direction of the belt.

The belt is preferably broad, to provide support in the abdominal region to prevent hernia of the abdominal wall, and to provide wearing comfort. Thus it is likely to fold. The ribs prevent the belt from folding and thus maintain its support function and comfort.

In an embodiment, the belt comprises two textile layers and an intermediate waterproof layer. This prevents fluids from leaking through the belt.

In an embodiment the intermediate layer comprises a rubber layer.

The rubber layer can be vulcanized or cross linked between the textile layers.

In an embodiment, the textile layers have anisotropic elasticity modulus, wherein a ratio of an elasticity modulus in the end-to-end direction relative to an elasticity modulus in the transverse direction is greater than 1. The elasticity in the end-to-end direction is as low as possible, preventing stretching in this direction. Preferably, the ratio is greater than 2. More preferably, the ratio is greater than 5.

### BRIEF DESCRIPTION OF THE DRAWINGS

Fig. 1 shows an isometric bottom view of an adapter for use in an ostomy pouch arrangement according to an embodiment the invention.
Fig. 2 shows a cross section of the ostomy pouch arrangement according to an embodiment the invention of fig. 1.
Fig. 3 shows an isometric bottom view of an adapter of an ostomy pouch arrangement according to an embodiment the invention.
Fig. 4 shows anisometric bottom view of an annular element of an ostomy pouch arrangement according to an embodiment the invention.
Fig. 5 shows a horizontal cross section of an ostomy pouch arrangement according to an embodiment the invention.
Fig. 6 shows a cross section of a detail of an adapter of an ostomy pouch arrangement according to an embodiment the invention.
Fig. 7a shows a top view of an ostomy pouch arrangement according to an embodiment the invention.
Fig. 7b shows a top view of an ostomy pouch arrangement according to an embodiment the invention.

### DETAILED DESCRIPTION OF EMBODIMENTS

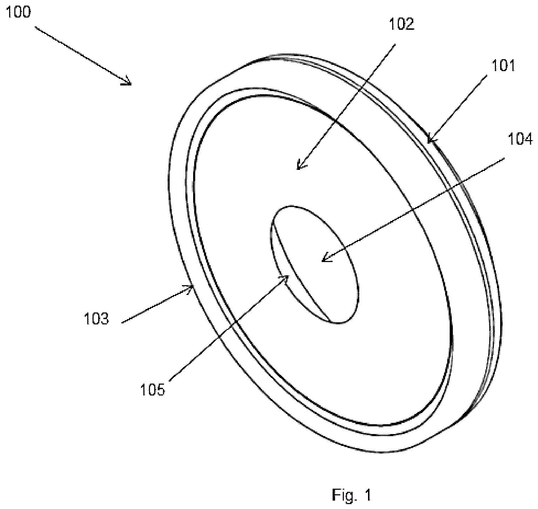

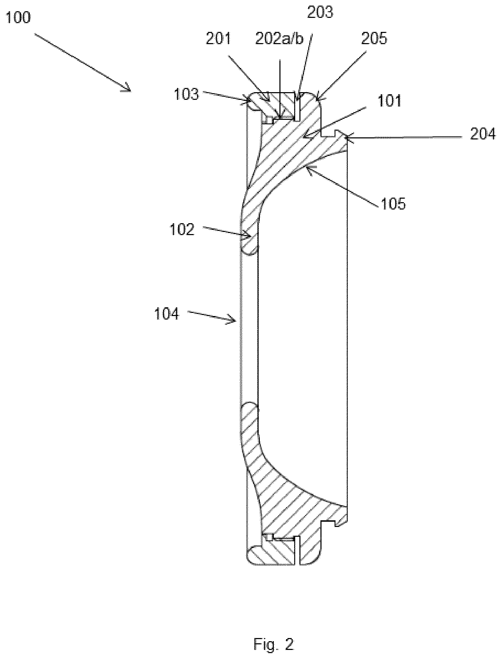

Fig. 1 shows an isometric bottom view of an adapter for use in an ostomy pouch arrangement according to the invention. The adapter 100 has an adapter body 101 which is cup-shaped having side walls 105 and a baseplate 102. The circumference of the baseplate is provided with a raised edge 103. In the center of the baseplate 102, an stoma receiving opening 104 is provided for receiving a stoma. The adapter 100 is designed to allow the baseplate 102 to rest against the subject's abdominal wall allowing the stoma to pass through the stoma receiving opening 104. The stoma receiving opening 104 can be manually adjusted to custom fit the subject's stoma. The shape of the stoma receiving opening 104 is shown circular in fig. 1, however the opening can have an arbitrary shape. The raised edge 103 rests in use against the subject's abdominal wall and prevents fluids from leaking from the space between the baseplate 102 and the subject's abdominal wall. The raised edge 103 is set off from the surface of the cup-shaped adapter body 101 facing the subject's abdominal wall 504. The raised edge 103 has a crest which may have a curved cross section, as shown in fig. 1 and 2 for comfortable use by the subject.

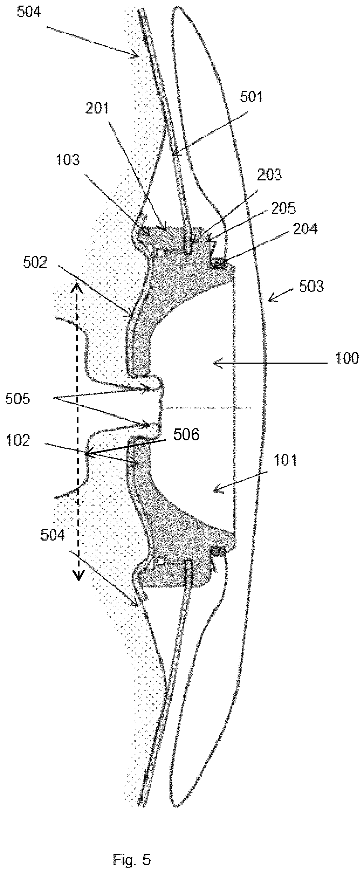

In fig. 2 a cross section of the adapter 100 from fig. 1 is shown, showing the cup-shaped adapter body 101, which is provided with an retention rim 204 for attaching and retaining a waist pouch for capturing excrements from the stoma. The retention rim 204 is shown by way of example. The skilled person will be aware of alternative solutions for attaching a waist pouch to the adapter body 101. The adapter body 101 has attached thereto an annular element 201 which is provided with the raised edge 103. The annular element 201 can be provided with a screw thread 202b for screwing unto a corresponding screw thread 202a of the lower portion of the adapter body 101. The annular element 201 can be screwed unto the adapter body up to flange 205 of the adapter body 101. Gap 203 between the annular element 201 and the flange 205 allows clamping of an edge of an opening 506 in the wearing belt 501 wherein the adapter 100 is to be fitted, it will be further depicted in fig. 5.

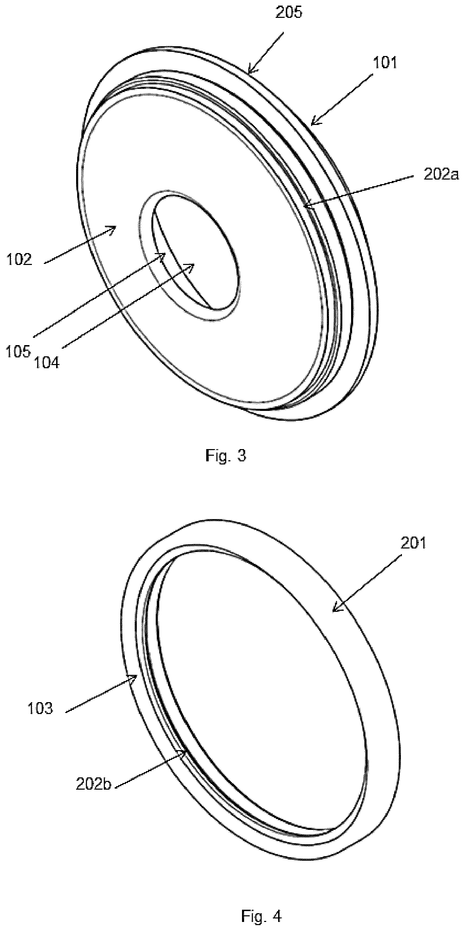

Fig. 3 shows a isometric bottom view of the adapter body 101. At the circumference of the baseplate 102 the screw thread 202a is shown for mounting the annular element 201 to the adapter body 101. Furthermore the flange 205 is shown up to which the annular element 201 is to be screwed using the screw thread 202a.

The screw connection 202a, 202b between the adapter body 101 and annular element 201 provides a removable connection. As an alternative to screwing, the annular element and adapter body 101 may be provided with cooperating edges between the adapter body 101 and annular element 201 in order to provide a snap fit. This provides a non-removable, permanent connection between the annular element 201 and the adapter body 101.

Fig. 4 shows an isometric bottom view of the annular element 201, wherein the raised edge 103 is shown and the screw thread 202b which is to cooperate with the screw thread 202a of the adapter body 101.

Fig. 5 shows a horizontal cross section of the adapter 100 as it is applied to a subject's abdominal wall 504. The adapter 100 rests with the baseplate 102 and the raised edge 103 against the subject's abdominal wall 504. A gauze pad 502 can be arranged between the adapter 100 and the subject's abdominal wall 504. The adapter 100 is arranged with stoma receiving opening 104 of the baseplate over the stoma 505. The adapter 100 is attached to the belt 501 with an edge of the belt opening, the belt opening depicted by dashed arrow with reference numeral 506, wherein the adapter is fitted clamped within the gap 203 between the annular element 201 and the flange 203. The belt 501 is arranged around the subject's body supporting the abdominal wall 504. In case of colostomy, the belt is usually worn around the subject's waist. Waste pouch 503 is attached to the retention rim 204 of the adapter body 101.

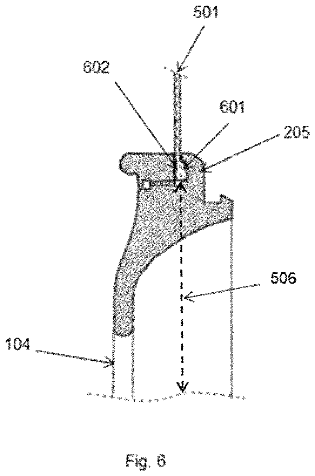

In fig. 6 a detail of the adapter 100 is shown, wherein the flange 205 is provided with an annular chamber 602 which allows a thickened edge 601 of the belt opening 506 to be locked in within the annular chamber 602. Alternatively, the annular chamber 602 may be provided within the top side of the annular element 201. Moreover both flange 205 and annular element 201 may be provided with a mutually corresponding annular chambers 602 for locking in the thickened edge 601 of the belt. The edge of the belt may be thickened for example by attaching a raised edge to that edge, or for example by stitching a hem onto that edge.

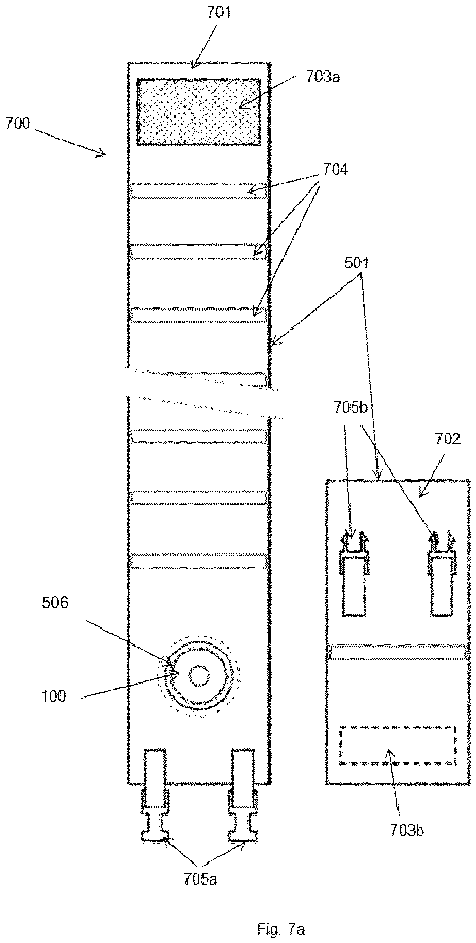

Fig. 7a shows a top view of an ostomy pouch arrangement 700. The arrangement 700 has a main belt section 701 which comprises the adapter 100 as described above, and a detachable and re-attachable section 702. The main belt section 701 and the detachable and re-attachable section 702 are connectable using fasteners 705a, 705b, and a hook and loop connection 703a, 703b.

The connection with fasteners 705a, 705b can be used by the subject wearing the ostomy pouch arrangement 700 for putting on the arrangement. The hook and loop connection 703a, 703b allow length adjustment of the belt 501 prior to actually putting on the arrangement 700, as well as adjustment of an angle between the belt main section and the detachable and re-attachable section 702. This allows adaptation of the belt 501 for body proportions of the wearer which may vary in time.

Alternatively, for the hook and loop connection 703a, 703b, length adjustment can be performed using for example straps with clasps or similar means.

The adapter 100 is fitted into belt opening 506 in the main belt section 701 near the fasteners 705a. This ensures that the fasteners 705a and 705b in use are located near the abdominal wall, as the adapter is required to be located at the abdominal wall as well. This contributes to the wearing comfort and puts the fasteners 705a, 705b well within reach of the subject wearing the arrangement 700.

The belt 501, i.e. main belt section 701 and detachable section are preferably made from a two textile layers having a waterproof intermediate layer for preventing fluids which may leak from the underneath the adapter 100 to stain the belt 501 and/or the subject's clothing. The waterproof layer can be rubber, vulcanized rubber, or a polymer material, such as polyurethane and polyurethane rubber, wherein the rubbers sustain the elasticity desired in the transverse direction of the belt 501. The layers can be attached to each other using a cross linking agent or adhesive. Optionally an absorption layer can be included between the textile layer facing the abdominal wall and the waterproof layer for absorbing leaked fluids. Moreover, the top textile layer facing the subject's clothing can be provided with a water proof or water repellent coating

The textile layers can be made from an anisotropic fabric having a high elasticity modulus (i.e. low or no stretch) in the end-to-end direction of the belt and a relatively low elasticity modulus (significant stretch) in the transverse direction. Thus it is achieved that the belt is rigid in the end-to-end direction , thereby providing adequate support for the abdominal wall, and resilient in the transverse direction, thereby providing comfort for the subject wearing the arrangement 700. The anisotropic property of the fabric can for example be achieved using fibers with high elasticity modulus in the end-to-end direction, and a low elasticity modulus in the transverse direction.

The main belt section 701 and detachable and re-attachable section 702 can be provided with ribs 704 which extend in a transverse direction relative to the end-to-end direction of the belt. The ribs 704 can be made from any rigid material, such as metal, plastic, wood, etc.. The ribs 704 can be accommodated within pockets attached to the belt sections 701, 702.

The belt width is chosen wide enough to provide hernia support. A width of approximately 8 cm or more can provide this support. The width of the belt however can vary over the end-to-end length of the belt as is known for hernia support belts. Moreover, the width of the belt can be adjusted depending on the wearer's requirements.

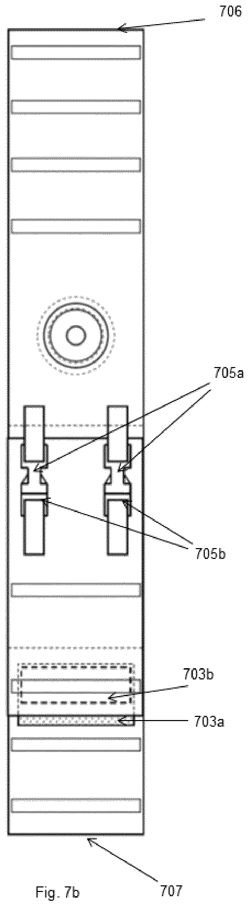

Fig. 7b shows a top view of the ostomy pouch arrangement 700 of fig. 7a, in an assembled state, i.e. the fasteners 705a, 705b and the hook and loop connection 703a, 703b are in a closed state. The belt 501 loops back from the top location 706 to bottom location 707 in fig. 7b.

It will be clear to a person skilled in the art that the scope of the present invention is not limited to the examples discussed in the foregoing but that several amendments and modifications thereof are possible without deviating from the scope of the present invention as defined by the attached claims. In particular, combinations of specific features of various aspects of the invention may be made. An aspect of the invention may be further advantageously enhanced by adding a feature that was described in relation to another aspect of the invention. While the present invention has been illustrated and described in detail in the figures and the description, such illustration and description are to be considered illustrative or exemplary only, and not restrictive.

The present invention is not limited to the disclosed embodiments. Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference numerals in the claims should not be construed as limiting the scope of the present invention.

### REFERENCE NUMERALS

- 100: adapter
- 101: adapter body
- 102: baseplate
- 103: raised edge
- 104: stoma receiving opening
- 105: side wall
- 201: annular element
- 202a, 202b: screw thread
- 203: gap
- 204: retention rim
- 205: flange
- 501: belt
- 502: gauze pad
- 503: waste pouch
- 504: abdominal wall
- 505: stoma
- 506: belt opening
- 601: thickened edge
- 602: annular chamber
- 701: main belt section
- 702: detachable and re-attachable belt section
- 703a, 703b: hook/loop element
- 704: rib
- 705a, 705b: fastener
- 706: top location
- 707: bottom location

## Claims

1. Ostomy pouch arrangement (700), comprising
• an adapter (100) having a cup shaped body (101) with an annular side wall (105) and a baseplate (102),
• wherein the baseplate (102) of the adapter (100) has an opening (104) for receiving a stoma (505);
• a belt (501) for securing the arrangement (700) on a subject's body, connected to the adapter (100) using connecting means (201, 202a, 202b);
• the adapter (100) has an interface (204) opposite the baseplate (102) for connecting an ostomy waste pouch (503);
• fastening means (705a, 705b) for tightening the belt around the subject's body;
• a continuous raised edge (103) at a circumference of the baseplate (102) of the adapter (100) in use in a direction facing a subject's abdominal wall (504),
wherein the adapter (100) has a flange (205) at the outside of the adapter body (101), and wherein the belt (501) has an opening (506) for fitting the adapter (100) to the belt (501), **characterized in that**
• the connecting means (201, 202a, 202b) comprise an annular element (201) which is attachable to the adapter body (101) at the side of the baseplate (102) of the adapter, in use facing the subject's abdominal wall (504), wherein the annular element (201) is arranged for clamping the edge (601) of the belt opening (506) against the flange (205).

2. Arrangement (700) according to claim 1, wherein the adapter body (101) of the adapter (100) is provided with a screw thread (202a), and the annular element (201) is provided with a screw thread (202b) corresponding with the screw thread (202a) of the adapter body (101).

3. Arrangement (700) according to claim 1 or claim 2, wherein the continuous raised edge (103) is arranged at the annular element (201) circumference.

4. Arrangement (700) according to any one of the preceding claims, wherein the edge (601) of the belt at the opening in the belt (506) has a thickening, and wherein at least one of the annular element (201) and the flange (205) have an annular chamber (601) for locking in the thickened edge (602) of the belt.

5. Arrangement (700) according to any one of the preceding claims, wherein the baseplate (102) of the adapter (100) has a convex shape.

6. Arrangement (700) according to any one of the claims 1 - 4, wherein the baseplate (102) of the adapter (100) has a flat shape.

7. Arrangement (700) according to any one of the claims 1 - 4, wherein the baseplate (102) of the adapter (100) has a concave shape.

8. Arrangement (700) according to any one of the preceding claims, wherein the belt (501) has two ends, in use facing each other, wherein each end is provided with corresponding mutually cooperating fastening means (705a, 705b) for fastening the two ends together.

9. Arrangement (700) according to claim 8, wherein the belt (501) has an opening (506) having the adapter (100) is positioned in proximity of one end having fastening means (705a).

10. Arrangement (700) according to claim 9, wherein the opposite end of the belt (501) has a detachable and re-attachable section (702) having the mutually cooperating fastening means (705a) for fastening the two ends together.

11. Arrangement (700) according to any one of the preceding claims, wherein the belt (501) is provided with ribs (704), which ribs (704) extend in a direction transverse to an end to end direction of the belt (501).

12. Arrangement (700) according to any one of the preceding claims, wherein the belt (501) comprises two textile layers and an intermediate waterproof layer.

13. Arrangement (700) according to claim 12, the intermediate layer comprises a rubber layer.

14. Arrangement (700) according to any one of the claims 12 - 13, wherein at least one of the textile layers have anisotropic elasticity, wherein a ratio of an elasticity modulus in the end-to-end direction relative to an elasticity modulus in the transverse direction is greater than 1.

## Patentansprüche

1. Ostomiebeutelanordnung (700), umfassend
• einen Adapter (100), der einen becherförmigen Körper (101) mit einer ringförmigen Seitenwand (105) und einer Bodenplatte (102) aufweist,
• wobei die Bodenplatte (102) des Adapters (100) eine Öffnung (104) zur Aufnahme eines Stomas (505) aufweist;
• einen Gürtel (501), um die Anordnung (700) am Körper eines Patienten zu befestigen, der am Adapter (100) mithilfe von Verbindungsmitteln (201, 202a, 202b) angeschlossen ist;
• der Adapter (100) weist eine Schnittstelle (204) gegenüber der Bodenplatte (102) für den Anschluss eines Ostomie-Abfallbeutels (503) auf;
• Befestigungsmittel (705a, 705b) zum Festziehen des Gürtels um den Körper des Patienten;
• eine durchgehende erhöhte Kante (103) am Umfang der Bodenplatte (102) des Adapters (100), bei Benutzung in einer Richtung die auf die Bauchwand (504) eines Patienten gerichtet ist,
wobei der Adapter (100) einen Flansch (205) an der Außenseite des Adapterkörpers (101) aufweist, und wobei der Gürtel (501) eine Öffnung (506) zum Anbringen des Adapters (100) am Gürtel (501) aufweist,
**dadurch gekennzeichnet, dass**
• die Verbindungsmittel (201, 202a, 202b) ein ringförmiges Element (201) umfassen, das am Adapterkörper (101) an der Seite der Bodenplatte (102) des Adapters befestigt werden kann, der bei Benutzung auf die Bauchwand des Patienten (504) gerichtet ist, wobei das ringförmige Element (201) angeordnet ist, um die Kante (601) der Gürtelöffnung (506) gegen den Flansch (205) zu klemmen.

2. Anordnung (700) nach Anspruch 1, wobei der Adapterkörper (101) des Adapters (100) mit einem Schraubengewinde (202a) versehen ist und das ringförmige Element (201) mit einem Schraubengewinde (202b) versehen ist, das dem Schraubengewinde (202a) des Adapterkörpers (101) entspricht.

3. Anordnung (700) nach Anspruch 1 oder Anspruch 2, wobei die durchgehende erhöhte Kante (103) am Umfang des ringförmigen Elements (201) angeordnet ist.

4. Anordnung (700) nach einem der vorhergehenden Ansprüche, wobei die Kante (601) des Gürtels an der Öffnung des Gürtels (506) eine Verdickung aufweist, und wobei mindestens eins von ringförmigem Element (201) und Flansch (205) eine ringförmige Kammer (601) zum Einrasten in der verdickten Kante (602) des Gürtels aufweist.

5. Anordnung (700) nach einem der vorhergehenden Ansprüche, wobei die Bodenplatte (102) des Adapters (100) eine konvexe Form aufweist.

6. Anordnung (700) nach einem der Ansprüche 1 - 4, wobei die Bodenplatte (102) des Adapters (100) eine flache Form aufweist.

7. Anordnung (700) nach einem der Ansprüche 1 - 4, wobei die Bodenplatte (102) des Adapters (100) eine konkave Form aufweist.

8. Anordnung (700) nach einem der vorhergehenden Ansprüche, wobei der Gürtel (501) zwei Enden hat, die bei Benutzung sich gegenüberstehen, wobei jedes Ende mit sich entsprechenden, zusammenwirkenden Befestigungsmitteln (705a, 705b) versehen ist, um die zwei Enden aneinander zu befestigen.

9. Anordnung (700) nach Anspruch 8, wobei der Gürtel (501) eine Öffnung (506) mit dem Adapter (100) aufweist, die in der Nähe von einem Ende mit Befestigungselement (705a) positioniert ist.

10. Anordnung (700) nach Anspruch 9, wobei das entgegengesetzte Ende des Gürtels (501) einen abnehmbaren und wieder befestigbaren Abschnitt (702) aufweist, der die zusammenwirkenden Befestigungsmittel (705a) aufweist, um die zwei Enden aneinander zu befestigen.

11. Anordnung (700) nach einem der vorhergehenden Ansprüche, wobei der Gürtel (501) mit Rippen (704) versehen ist, wobei sich die Rippen (704) in einer Richtung quer zu einer Richtung von Ende zu Ende des Gürtels (501) erstrecken.

12. Anordnung (700) nach einem der vorhergehenden Ansprüche, wobei der Gürtel (501) zwei Gewebeschichten und eine wasserdichte Zwischenschicht umfasst.

13. Anordnung (700) nach Anspruch 12, die Zwischenschicht umfasst eine Gummischicht.

14. Anordnung (700) nach einem der Ansprüche 12 - 13, wobei mindestens eine der Gewebeschichten anisotrope Elastizität aufweist, wobei das Verhältnis eines Elastizitätsmoduls in der von Ende zu Ende Richtung zu einem Elastizitätsmodul in der Querrichtung größer als 1 ist.

## Revendications

1. Dispositif de poche de stomie (700), comprenant
• un adaptateur (100) comportant un corps en forme de coupe (101) doté d'une paroi latérale annulaire (105) et d'une plaque de base (102),
• dans lequel la plaque de base (102) de l'adaptateur (100) comporte une ouverture (104) destinée à recevoir une stomie (505),
• une ceinture (501) destinée à fixer le dispositif (700) sur le corps d'un sujet et connectée par l'adaptateur (100) en utilisant des moyens de connexion (201, 202a, 202b) ;
• l'adaptateur (100) comporte une interface (204) opposée à la plaque de base (102) pour la connexion d'une poche de déchets de stomie (503);
• des moyens de fixation (705a, 705b) pour serrer la ceinture autour du corps du sujet ;
• un bord surélevé continu (103) au niveau d'une circonférence de la plaque de base (102) de l'adaptateur (100) en cours d'utilisation dans une direction faisant face à la paroi abdominale du sujet (504),
l'adaptateur (100) comportant une bride (205) sur l'extérieur du corps d'adaptateur (100), et la ceinture (501) comportant une ouverture (506) pour ajuster l'adaptateur (100) à la ceinture (501),
**caractérisé en ce que**
• le moyen de connexion (201, 202a, 202b) comprend un élément annulaire (201) qui peut être rattaché au corps d'adaptateur (101) sur le côté de la plaque de base (102) de l'adaptateur en cours d'utilisation face à la paroi abdominale du sujet (504), l'élément annulaire (201) étant conçu pour serrer le bord (601) de l'ouverture de la ceinture (506) contre la bride (205).

2. Dispositif (700) selon la revendication 1, dans lequel le corps d'adaptateur (101) de l'adaptateur (100) est pourvu d'un filetage (202a), et l'élément annulaire (201) est pourvu d'un filetage (202b) correspondant au filetage (202a) du corps d'adaptateur (101).

3. Dispositif (700) selon la revendication 1 ou la revendication 2, dans lequel le bord surélevé continu (103) est disposé au niveau de la circonférence de l'élément annulaire (201).

4. Dispositif (700) selon l'une quelconque des revendications précédentes, dans lequel le bord (601) de la ceinture au niveau de l'ouverture dans la ceinture (506) comprend un épaississement et au moins un parmi l'élément annulaire (201) et la bride (205) comporte une chambre annulaire (601) pour verrouiller le bord épaissi (602) de la ceinture.

5. Dispositif (700) selon l'une quelconque des revendications précédentes, dans lequel la plaque de base (102) de l'adaptateur (100) a une forme convexe.

6. Dispositif (700) selon l'une quelconque des revendications 1 à 4, dans lequel la plaque de base (102) de l'adaptateur (100) a une forme plate.

7. Dispositif (700) selon l'une quelconque des revendications 1 à 4, dans lequel la plaque de base (102) de l'adaptateur (100) a une forme concave.

8. Dispositif (700) selon l'une quelconque des revendications précédentes, dans lequel la ceinture (501) a deux extrémités se faisant mutuellement face en utilisation, chaque unité étant pourvue de moyens de fixation se correspondant mutuellement (705a, 705b) pour fixer les deux extrémités ensemble.

9. Dispositif (700) selon la revendication 8, dans lequel la ceinture (501) ayant une ouverture (506) comportant l'adaptateur (100), étant positionné à proximité d'une extrémité comportant des moyens de fixation (705a).

10. Dispositif (700) selon la revendication 9, dans lequel l'extrémité opposée de la ceinture (501) comporte une section détachable et rattachable (702) comportant les moyens de fixation coopérant mutuellement (705a) pour fixer les deux extrémités ensemble.

11. Dispositif (700) selon l'une quelconque des revendications précédentes, dans lequel la ceinture (501) est pourvue de nervures (704), lesquelles nervures (704) s'étendent dans un sens transversal à un sens d'extrémité à extrémité de la ceinture (501).

12. Dispositif (700) selon l'une quelconque des revendications précédentes, dans lequel la ceinture (501) comprend deux couches de textile et une couche imperméable intermédiaire.

13. Dispositif (700) selon la revendication 12, dans lequel la couche intermédiaire comprend une couche de caoutchouc.

14. Dispositif (700) selon l'une quelconque des revendications 12 - 13, dans lequel au moins une des couches de textile a une élasticité anisotrope, un rapport entre un module d'élasticité dans le sens d'extrémité à extrémité et un module d'élasticité dans le sens transversal étant supérieur à 1.
